# EUROPEAN PATENT APPLICATION

(11) **EP 4 273 244 A1**
(43) Date of publication of application: **08.11.2023**
(21) Application number: 22756228.7
(22) Date of filing: 16.02.2022
(51) Int. Cl.: C12N 15/11, C07K 16/00, C12Q 1/686, C12Q 1/6865, C12Q 1/6869

(54) **NUCLEIC ACID FRAGMENT AND USE THEREOF**

(30) Priority: 19.02.2021 JP 2021025468
(71) Applicant: Kyushu University, National University Corporation, Nishi-ku Fukuoka-shi Fukuoka 819-0395 (JP)
(72) Inventor: OHKAWA Yasuyuki, Fukuoka-shi, Fukuoka 819-0395 (JP); HARADA Akihito, Fukuoka-shi, Fukuoka 819-0395 (JP); OKI Shinya, Fukuoka-shi, Fukuoka 819-0395 (JP)
(74) Representative: EP&C
(86) International application number: PCT/JP2022/006187
(87) International publication number: WO 2022/176910

(57) **Abstract**

A nucleic acid fragment including a polymerase binding sequence and a transposase binding sequence, in which the polymerase binding sequence or the transposase binding sequence contains a caged nucleotide residue; a specific binding substance; a method for binding a polymerase or a transposase to the nucleic acid fragment; a method for inserting the nucleic acid fragment in a vicinity of a binding region of a DNA-binding protein bound to a DNA molecule; and a method for gene-amplifying the DNA molecule into which the nucleic acid fragment is inserted are provided.

## Description

### [Technical Field]

The present invention relates to a nucleic acid fragment and use thereof. More specifically, the present invention relates to a nucleic acid fragment, a specific binding substance, a method for binding a polymerase or a transposase to a nucleic acid fragment, a method for inserting a nucleic acid fragment into the vicinity of a binding region of a DNA-binding protein bound to a DNA molecule, and a method for gene-amplifying a DNA molecule into which a nucleic acid fragment is inserted. Priority is claimed on Japanese Patent Application No. 2021-025468, filed February 19, 2021, the content of which is incorporated herein by reference.

### [Background Art]

There is a demand for detecting a base sequence of a target nucleic acid present in each of a plurality of cells at the level of one cell. For example, the inventors previously developed a technique for analyzing binding of a DNA-binding protein to a specific genomic region at the level of one cell (refer to Patent Document 1).

In a case of the method disclosed in Patent Document 1, the base sequence of the target nucleic acid is a base sequence in the vicinity of a binding region of a DNA-binding protein on a genomic DNA. According to the method disclosed in Patent Document 1, a binding position of the DNA-binding protein on the genomic DNA can be analyzed at the level of one cell.

### [Citation List]

### [Patent Document]

[Patent Document 1]
PCT International Publication No. WO2018/042776

### [Summary of Invention]

### [Technical Problem]

However, in order to carry out the method disclosed in Patent Document 1 at the level of one cell, it is necessary to cut out target cells one by one by laser microdissection and the like. An object of the present invention is to provide a simpler technique capable of locally analyzing a base sequence of a target nucleic acid, for example, at the level of one cell or at the level of a sub-cell.

### [Solution to Problem]

The present invention includes the following aspects.
[1] A nucleic acid fragment including a polymerase binding sequence and a transposase binding sequence, in which the polymerase binding sequence of the transposase binding sequence contains a caged nucleotide residue.
[2] The nucleic acid fragment according to [1], which is a double-stranded nucleic acid fragment.
[3] The nucleic acid fragment according to [1] or [2], in which a caging group of the caged nucleotide residue contained in the polymerase binding sequence and a caging group of the caged nucleotide residue contained in the transposase binding sequence are different groups.
[4] A specific binding substance for a target molecule labeled with the nucleic acid fragment according to any one of [1] to [3].
[5] A method for binding a polymerase or a transposase to the nucleic acid fragment according to any one of [1] to [3], the method including: a step of irradiating the nucleic acid fragment with active energy rays and eliminating a caging group from the caged nucleotide residue; and a step of bringing the polymerase or the transposase into contact with the nucleic acid fragment.
[6] A method for inserting a nucleic acid fragment containing a polymerase binding sequence and a transposase binding sequence in a vicinity of a binding region of a DNA-binding protein bound to a DNA molecule, the transposase binding sequence containing a caged nucleotide residue, the method including: a step of bringing the nucleic acid fragment and the binding region close to each other by using a specific binding substance for the DNA-binding protein; a step of irradiating the nucleic acid fragment with active energy rays and eliminating a caging group from the caged nucleotide residue of the transposase binding sequence; a step of binding a transposase to the transposase binding sequence from which the caging group is eliminated; and a step of activating the transposase, and as a result, inserting the nucleic acid fragment in the vicinity of the binding region.
[7] A method for gene-amplifying a DNA molecule, the DNA molecule containing a polymerase binding sequence and a transposase binding sequence, in which the polymerase binding sequence contains a caged nucleotide residue, and into which a nucleic acid fragment is inserted, starting from the polymerase binding sequence, the method including a step of irradiating the nucleic acid fragment with active energy rays and eliminating a caging group from the caged nucleotide residue of the polymerase binding sequence, and a step of binding a polymerase to the polymerase binding sequence from which the caging group is eliminated to obtain an amplification product.

### [Advantageous Effects of Invention]

According to the present invention, it is possible to provide a simpler technique capable of locally analyzing a base sequence of a target nucleic acid, for example, at the level of one cell or at the level of a sub-cell.

### [Brief Description of Drawings]

FIG. 1 is a schematic diagram showing an example of a chromatin integration labeling method (ChIL method).
FIG. 2 is a schematic diagram showing the ChIL method.
FIG. 3 is a schematic diagram showing the ChIL method.
FIG. 4 is a schematic diagram showing the ChIL method.
FIG. 5(a) to FIG. 5(c) are schematic diagrams showing the ChIL method.
FIG. 6(a) and FIG. 6(b) are schematic diagrams showing the ChIL method.
FIG. 7 is a photograph showing results of Experimental Example 1.
FIG. 8 is a photograph showing results of agarose electrophoresis in Experimental Example 2.
FIG. 9 is a diagram showing results of visualizing reads obtained by next generation sequencing in Experimental Example 2.

### [Description of Embodiments]

Hereinafter, embodiments of the present invention will be described in detail with reference to the drawings depending on the case. In the drawings, the same of corresponding parts are denoted by the same or corresponding reference numerals, and overlapping descriptions are omitted. Note that dimensional ratios in each drawing are exaggerated for the sake of explanation, and do not necessarily match the actual dimensional ratios.

### [ChIL method]

First, a chromatin integration labeling (ChIL method) will be described. The ChIL method is a method for inserting a DNA fragment of a desired base sequence in the vicinity of a binding region of a DNA-binding protein bound to a DNA molecule, using a transposase.

The ChIL method is a method for inserting a DNA fragment of a desired base sequence in the vicinity of a binding region of a DNA-binding protein bound to a DNA molecule, and includes a step (a) of bringing a DNA fragment having a base sequence including a transposase binding sequence and the desired base sequence and the binding region close to each other using a specific binding substance to the DNA-binding protein; a step (b) of binding a transposase to the transposase binding sequence; and a step (c) of activating the transposase and, as a result, inserting the DNA fragment of the desired base sequence in the vicinity of the binding region.

According to the ChIL method, binding of a DNA-binding protein to a specific genomic region can be analyzed without performing immunoprecipitation. Therefore, according to the ChIL method, it is possible to analyze the binding of the DNA-binding protein, which was difficult to analyze in the related art in which immunoprecipitation is an essential step, to a DNA. In addition, in the related art, it was difficult to analyze DNA-binding proteins at the level of one cell. On the other hand, according to the ChIL method, it is possible to perform analysis on DNA-binding proteins at the level of one cell.

The DNA-binding protein to be analyzed is not particularly limited, and examples thereof include histones, transcription factors, phosphorylated polymerases, and the like. For example, a transcription state of a gene can be analyzed by analyzing an RNA polymerase II in a specific phosphorylation state as a DNA-binding protein.

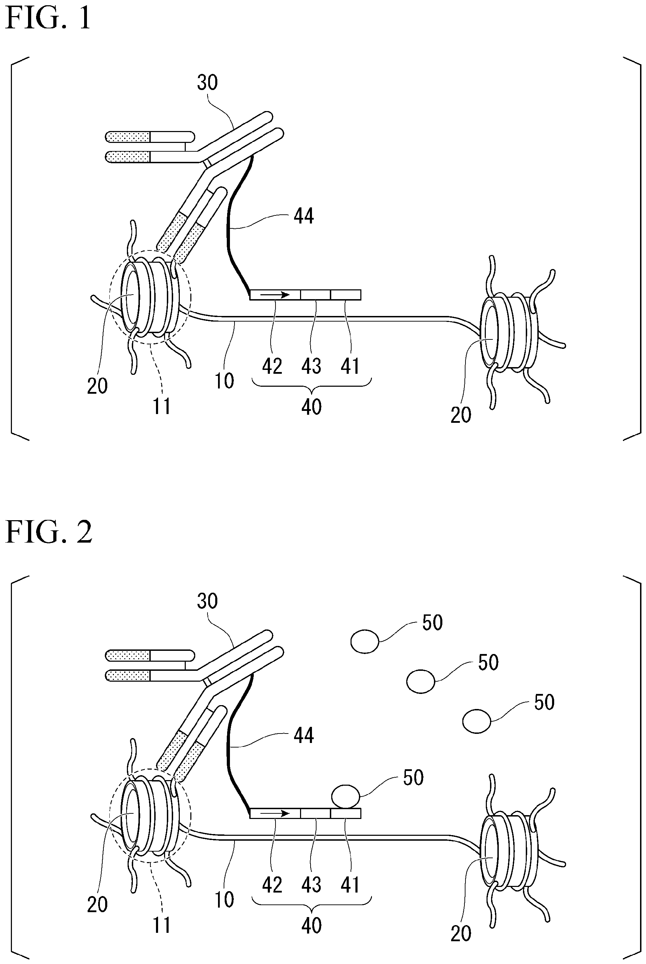

Hereinafter, the ChIL method will be described with reference to FIGS. 1 to 6. FIGS. 1 to 6 are schematic diagrams describing the ChIL method. FIG. 1 shows a state in which a genomic DNA 10 is wound around a histone 20 to form a chromatin structure. In the example of FIG. 1, a DNA-binding protein is a histone 20.

In the example of FIG. 1, a specific binding substance 30 is an antibody that recognizes a histone 20. A DNA fragment 40 is bound to the specific binding substance 30 via a linker 44. The DNA fragment 40 has a base sequence including a transposase binding sequence 41 and a desired base sequence. In the example of FIG. 1, the desired base sequence is a base sequence in which an identification sequence 43 is linked downstream of a T7 promoter sequence 42. In FIGS. 1 to 6, an arrow of the T7 promoter sequence 42 indicates an orientation of transcription by a T7 polymerase.

The linker 44 has the role of a tether to bring the DNA fragment 40 close to a binding region 11 of the histone 20. In the example of FIG. 1, the linker 44 is a DNA fragment and has a length of, for example, 50 to 70 bases. However, the linker 44 is not particularly limited as long as it functions as a tether, and may be constituted of a polymer such as polyethylene glycol.

### (Step (a))

In the example of FIG. 1, the ChIL method is carried out using cells fixed with paraformaldehyde as a sample. First, the DNA fragment 40 and the binding region 11 are brought close to each other by using the specific binding substance 30 to the histone 20. Here, that the specific binding substance 30 is used to bring the DNA fragment 40 and the binding region 11 close to each other may mean that the specific binding substance 30 to which the DNA fragment 40 is bound as in the example of FIG. 1 is bound to the histone 20, which is an antigen thereof, thereby bringing the DNA fragment 40 and the binding region 11 close to each other. Alternatively, the specific binding substance 30 is, for example, a mouse IgG antibody against the histone 20, and by binding the mouse IgG antibody as a primary antibody to the histone 20, and subsequently binding an anti-mouse IgG antibody to which the DNA fragment 40 is bound to the primary antibody as a secondary antibody, the DNA fragment 40 and the binding region 11 may be brought close to each other.

A step of bringing the DNA fragment 40 and the binding region 11 close to each other using the specific binding substance 30 to the histone 20 can be carried out in a fixed cell, as in the example of FIG. 1. In this step, it is also possible to use a plurality of antibodies simultaneously, as in immunostaining. Therefore, according to the ChIL method, DNA fragments having different desired base sequences can be inserted in the vicinity of the binding region of a plurality of DNA-binding proteins. Here, by including a distinguishable sequence for each DNA-binding protein in the identification sequence 43, the binding of a plurality of DNA-binding proteins to DNA can be distinguished and detected.

### (Step (b))

Subsequently, a transposase 50 is bound to a transposase binding sequence 41. FIG. 2 shows a state in which the transposase 50 is added to a sample and the transposase 50 is bound to the transposase binding sequence 41.

The transposase is preferably small in size from the viewpoint of easy entry into the nucleus. For example, a transposase having a molecular weight of 50 KDa or less is preferable. In addition, sequence specificity of the recognized sequence is preferably high. In addition, a DNA-type transposase is preferable. In addition, a transposase capable of controlling the activity is preferable, and examples thereof include a transposase that is activated in a case where a cation such as a divalent metal ion is added to a buffer. Preferable examples of the transposase include TN5 transposase, sleeping beauty transposase (SB10), TN10 transposase, and the like. The transposase binding sequence 41 may be determined depending on the used transposase.

### (Step (c))

Subsequently, the above transposase 50 is activated. Activation of the transposase 50 can be carried out, for example, by adding a divalent metal ion to a buffer.

In addition, depending on the type of the transposase 50, there is a case where it is necessary to form a dimer in order to exhibit the transposition activity of a DNA fragment. In addition, in order for the transposase 50 to form a dimer, there is a case where it may be necessary to be bind the transposase 50 to the transposase binding sequence 41. For example, TN5, SB10, TN10, and the like described above are such transposases. In such a case, the transposase 50 can also be activated by forming a dimer of the transposase 50.

In addition, depending on the type of the transposase 50, in order to activate the transposase 50, there is a case where it is necessary to form a dimer of the transposase 50 and to add a divalent metal ion to a buffer.

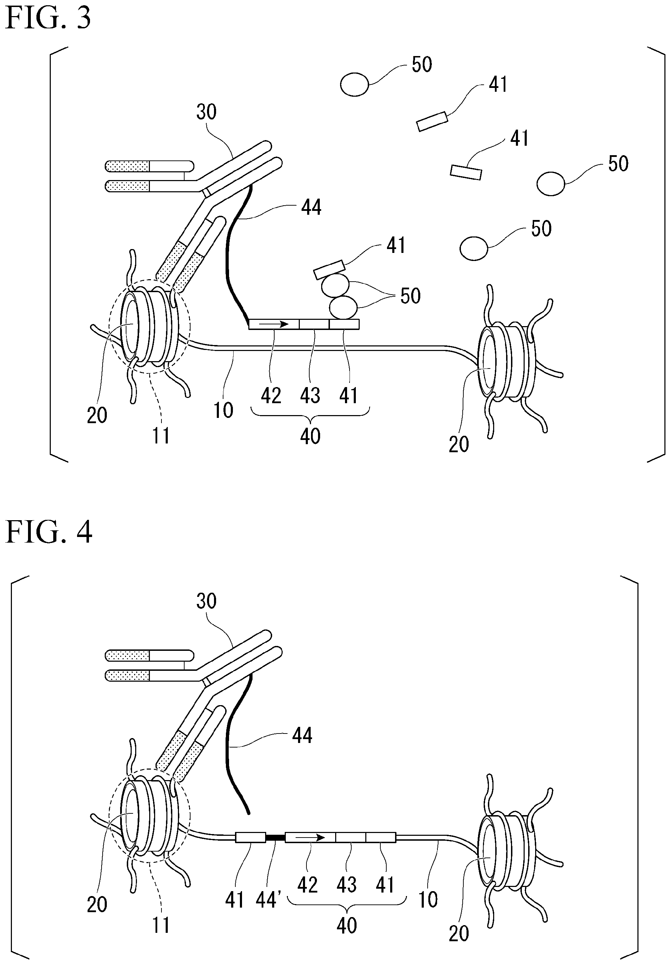

FIG. 3 shows a state in which the dimer of the transposase 50 is formed. In FIG. 3, a free DNA fragment having the transposase binding sequence 41 is added to a sample, and the transposase 50 is bound to the DNA fragment. As shown in FIG. 3, the transposase 50 bound to the above DNA fragment forms a dimer with the transposase 50 bound to the transposase binding sequence 41 constituting the DNA fragment 40.

As a result of activating the transposase 50, the DNA fragment of a desired base sequence (a base sequence in which the identification sequence 43 is linked downstream of the T7 promoter sequence 42 in the example of FIG. 3) in the DNA fragment 40 is inserted in the vicinity of the binding region 11. Here, the vicinity may be a region that is spatially close, and may be, for example, 500 bases or less from the binding region 11, for example, 100 bases or less, or, for example, 50 bases or less.

Alternatively, since the vicinity may be a region that is spatially close, the vicinity may be a region that is significantly far from the binding region 11 on the base sequence of the genomic DNA 10 and may be a region that is close due to an interaction between genomic regions. That is, the region may be a region that is close in terms of a chromosome structure.

In the present step, there is a case where the insertion reaction of the DNA fragment by the transposase 50 may be incompletely terminated. Specifically, examples include a case where the inserted DNA fragment is partially a single-stranded DNA, a case where the inserted DNA fragment is fragmented, and the like. Therefore, it is preferable to further carry out the step of completely terminating the insertion reaction of the DNA fragment by the transposase 50 by a fill-in reaction and the like using T4DNA ligase, T4DNA polymerase, and the like.

FIG. 4 is a schematic diagram showing an example of a state in which a DNA fragment of a desired base sequence is inserted in the vicinity of the binding region 11. As shown in FIG. 4, in this example, a DNA fragment of a desired base sequence (a base sequence in which an identification sequence 43 is linked downstream of a T7 promoter sequence 42) is inserted in the vicinity of the binding region 11. In the example of FIG. 4, not only a desired base sequence but also a base sequence 44' derived from a part of two transposase binding sequences 41 and the linker 44 is inserted into the genomic DNA 10.

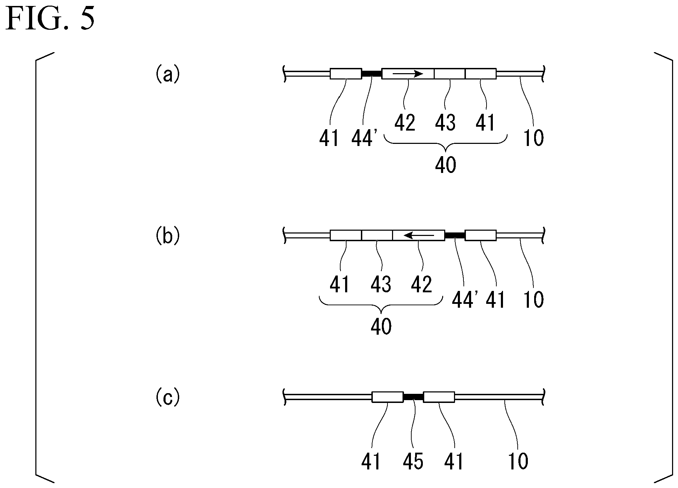

FIGS. 5(a) to 5(c) are schematic diagrams showing an example of a case where a DNA fragment is inserted into a genomic DNA 10 as a result of activation of the transposase 50 in a state of the above-described FIG. 2. FIG. 5(a) shows a state of the above-described FIG. 4. FIG. 5(b) shows a state in which a desired base sequence is inserted in a reverse orientation of the above-described FIG. 4. FIG. 5(c) shows a base sequence that can be inserted into the genomic DNA 10 in a case where the transposases 50 bound to a free DNA fragment having the transposase binding sequence 41 form a dimer.

There is a case where the base sequence inserted into the genomic DNA 10 in FIG. 5(c) includes two transposase binding sequences 41 and includes an optional base sequence 45 therebetween. The base sequence 45 is formed by the above-described fill-in reaction.

The base sequence shown in FIG. 5(c) is an unintended by-product and can be inserted into any part on the genomic DNA 10, and is not limited to the vicinity of the binding region 11. However, the base sequence shown in FIG. 5(c) does not have a desired base sequence (in this example, a base sequence in which the identification sequence 43 is linked downstream of the T7 promoter sequence 42). For this reason, it is possible to exclude any influence in the intended analysis.

Through the above steps, a DNA fragment of a desired base sequence can be inserted in the vicinity of a binding region of a DNA-binding protein bound to a DNA molecule.

### (Desired base sequence)

In addition, in the examples of FIGS. 1 to 5, the desired base sequence inserted into the genomic DNA 10 is the base sequence in which the identification sequence 43 is linked downstream of the T7 promoter sequence 42 as a polymerase binding sequence, but the desired base sequence is not limited thereto. For example, as a polymerase binding sequence, another promoter sequence such as an SP6 promoter sequence may be used instead of the T7 promoter sequence 42. Alternatively, the desired base sequence may be constituted of only the identification sequence.

The length of the desired base sequence inserted into the genomic DNA 10 may be, for example, 20 to 500 bases, 20 to 200 bases, or 20 to 100 bases.

In addition, in the examples of FIGS. 1 to 5, each element (T7 promoter sequence 42, identification sequence 43, and transposase binding sequence 41) constituting the DNA fragment 40 is adjacent, but a spacer of an optional base sequence may be present between each of these elements. In addition, the order of each of the elements is not limited to those shown in the examples of FIGS. 1 to 5, and may be appropriately replaced, or additional elements may be added depending on the necessity.

In addition, the DNA fragment 40 may be single-stranded or double-stranded. It is preferable that the DNA fragment 40 be double-stranded since a charge is neutralized and non-specific binding of the DNA fragment 40 to an unintended location can be suppressed. In addition, in a case where the transposase binding sequence 41 is required to be a double-stranded DNA due to characteristics of the used transposase, at least the transposase binding sequence 41 is required to be double-stranded.

### (Analysis using DNA fragment inserted by ChIL method)

The ChIL method includes a step of gene-amplifying a DNA 10 starting from a desired base sequence (a base sequence in which the identification sequence 43 is linked downstream of the T7 promoter sequence 42 in the examples of FIGS. 1 to 5) to obtain an amplification product and a step of analyzing the base sequence of the amplification product, and by the method, it is possible to analyze a position on a genome into which the desired DNA fragment is inserted.

### (Gene amplification)

Examples of gene amplification include PCR in which primers complementary to an inserted base sequence and random primers are combined, transcription using a polymerase (RNA polymerase, DNA-dependent DNA polymerase, and the like), and the like. In the present specification, the phrase "gene amplification starting from a desired base sequence" means gene amplification of the genomic DNA 10 by using a part or all of a desired base sequence inserted in the vicinity of the binding region 11.

For example, in the examples of FIGS. 1 to 5, the T7 promoter sequence 42 is included in the base sequence inserted into the genomic DNA 10. Therefore, the phrase "gene amplification starting from a desired base sequence" means that the genomic DNA 10 downstream of the T7 promoter sequence 42 may be transcribed into RNA by causing the T7 polymerase to act on the genomic DNA 10 to be transcribed.

Alternatively, the phrase "gene amplification starting from a desired base sequence" means that by performing PCR in which a primer having a base sequence complementary to a part or all of an inserted base sequence using the genomic DNA 10 as a template, and a random primer are combined and the like, the base sequence of the genomic DNA 10 in the vicinity of the binding region 11 may be gene-amplified.

That is, in the ChIL method, the inserted base sequence includes a promoter sequence, and gene amplification may be performed by bringing an RNA polymerase into contact with the promoter sequence and transcribing DNA downstream of the promoter sequence to generate RNA. As described above, since the base sequence shown in FIG. 5(c) does not have the T7 promoter sequence 42, the gene amplification (transcription) is not performed even in a case where the T7 polymerase is caused to act.

Alternatively, the gene amplification may be performed by PCR using an inserted desired base sequence and the like. As described above, since the base sequence shown in FIG. 5(c) does not have a desired base sequence (a base sequence in which the identification sequence 43 is bound downstream of the T7 promoter sequence 42), gene amplification is not performed even if a PCR reaction using the inserted base sequence is performed.

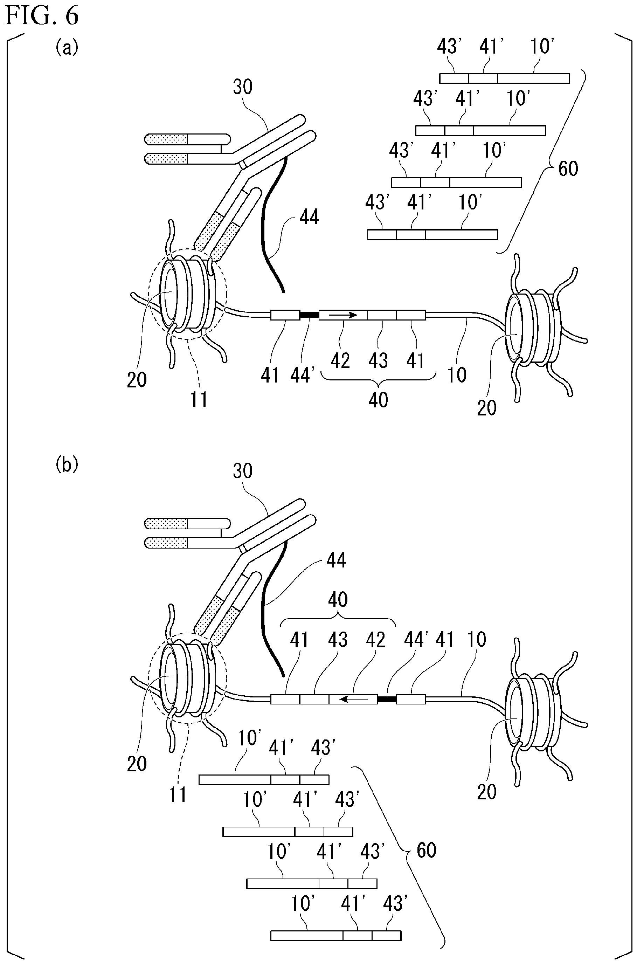

FIG. 6(a) is a diagram showing a state in which the DNA 10 downstream of the T7 promoter sequence 42 is transcribed into RNA 60 by causing the T7 polymerase to act on the DNA 10 shown in FIGS. 4 or 5(a). In addition, FIG. 6(b) is a diagram showing a state in which the DNA 10 downstream of the T7 promoter sequence 42 is transcribed into RNA 60 by causing the T7 polymerase to act on the DNA 10 shown in FIG. 5(b).

In the examples of FIGS. 6(a) and 6(b), since the identification sequence 43 is introduced downstream of the T7 promoter sequence 42, the RNA 60 includes a complementary strand 43' of the identification sequence 43 and a complementary strand 10' of the DNA 10 downstream of the identification sequence 43.

In a case where the T7 RNA polymerase is caused to act on the genomic DNA, there is a case where RNA may be transcribed from a region other than the T7 promoter sequence 42 inserted by the above reaction. On the other hand, as the identification sequence 43 is present, it is possible to identify the RNA transcribed starting from the inserted T7 promoter sequence 42 and an RNA other than the RNA. That is, it can be determined that RNA 60 having a complementary strand 43' of the identification sequence 43 is an RNA transcribed starting from the inserted T7 promoter sequence 42.

In addition, as described above, in the ChIL method, it is possible to simultaneously perform analysis using specific binding substances for a plurality of DNA-binding proteins. Here, by introducing different identification sequences 43 into the DNA fragment 40 to be inserted into the DNA 10 by using each specific binding substance, it is possible to identify the RNA transcribed from the vicinity of the binding region of any DNA-binding protein.

Subsequently, by analyzing the base sequence of the transcribed RNA 60, it is possible to specify a position on the genome to which the DNA-binding protein is bound. The analysis of the base sequence of RNA 60 may be performed by, for example, a next generation sequencer, hybridization with a DNA array, and the like.

In the examples of FIGS. 6(a) and 6(b) described above, regions transcribed into the RNA 60 are different from each other, but in any of the examples, the RNA 60 is an RNA transcribed from the vicinity of the binding region 11. Therefore, by analyzing the base sequence of the RNA 60, for example, it is possible to specify a position on the genome of the binding region of the DNA-binding protein (histone 20 in the examples of FIGS. 1 to 6). That is, the orientation in which a desired base sequence is inserted does not affect the analysis result.

### (Conjugate)

Here, a conjugate (ChIL probe) used in the ChIL method will be described. Here, the conjugate is a conjugate in which a DNA fragment having a base sequence including a transposase binding sequence and a desired base sequence, and a specific binding substance for a DNA-binding protein or a specific binding substance for the specific binding substance are bound.

In the conjugate, the transposase binding sequence, the desired base sequence, and the specific binding substance are the same as those described above. That is, the desired base sequence includes an identification sequence. In addition, the desired base sequence may further include a promoter sequence upstream of the identification sequence.

In the conjugate, the DNA fragment is preferably bound to a specific binding substance for a DNA-binding protein or a specific binding substance for the specific binding substance via a linker. The linker is the same as that described above.

The phrase "a conjugate of a desired DNA fragment and a specific binding substance for a DNA-binding protein" means, for example, a conjugate in which a desired DNA fragment is bound to a primary antibody. That is, the conjugate may be a conjugate in which the DNA fragment is directly bound to a specific binding substance for a DNA-binding protein.

In addition, the phrase "a conjugate of a desired DNA fragment and a specific binding substance for the specific binding substance for a DNA-binding protein" means, for example, a conjugate in which a desired DNA fragment is bound to a secondary antibody. That is, the desired DNA fragment may not be bound to a specific binding substance for a DNA-binding protein but may be bound to a specific binding substance that is bound to a specific binding substance for a DNA-binding protein. More specifically, examples thereof include a conjugate of a desired DNA fragment and an anti-mouse IgG antibody in a case where the specific binding substance for the DNA-binding protein is mouse IgG.

The number of the desired DNA fragments per molecule of the specific binding substance is not particularly limited, and can be, for example, about 1 to 10. In addition, the method for binding a DNA fragment to a specific binding substance is not particularly limited, and binding may be carried out using, for example, a chemical cross-linking agent having a succinimide group, a maleimide group, and the like. For example, an amino group may be bound to the 5'-terminal of a DNA fragment, and examples thereof include a covalent binding of the amino group and a functional group such as an amino group or a carboxy group present in the specific binding substance with an appropriate chemical cross-linking agent. Alternatively, a DNA fragment may be bound to a specific binding substance by using, for example, binding of avidin and biotin and the like.

### [Nucleic acid fragment]

In an embodiment, the present invention provides a nucleic acid fragment containing a polymerase binding sequence and a transposase binding sequence, in which the polymerase binding sequence or the transposase binding sequence includes a caged nucleotide residue. The nucleic acid fragment of the present embodiment may further contain an identification sequence. In this case, the identification sequence is preferably arranged to be transcribed by a polymerase bound to a polymerase binding sequence.

The nucleic acid fragment of the present embodiment can be suitably used, for example, in the above-described conjugate (ChIL probe). That is, the nucleic acid fragment of the present embodiment can be used as the DNA fragment having a base sequence including the transposase binding sequence and a desired base sequence described above. Therefore, it can be also said that the nucleic acid fragment of the present embodiment is a nucleic acid fragment for a ChIL probe.

By binding the nucleic acid fragment of the present embodiment to a specific binding substance for a DNA-binding protein or a specific binding substance for the specific binding substance to form a conjugate (ChIL probe) used in the ChIL method, the ChIL method can be locally carried out more conveniently than in the related art. Here, the term "locally" may be, for example, at the level of tens of cells, may be at the level of several cells, may be at the level of one cell, or may be at the level of a sub-cell.

In the nucleic acid fragment of the present embodiment, the polymerase binding sequence is the same as described above, and for example, a T7 promoter sequence, an SP6 promoter sequence, and the like can be used.

The identification sequence is not particularly limited as long as it is a base sequence having a low appearance frequency probabilistically, and for example, any base sequence of about 4 to 20 bases can be used. As a more specific identification sequence, for example, any of the base sequences shown in Tables 1 and 2 below can be used.

Table 1 below shows 384 base sequences. Table 2 below shows 324 base sequences. The base sequences shown in Tables 1 and 2 below are artificially created base sequences, and are designed such that at least two or more bases are different from each other in consideration of sequence errors.

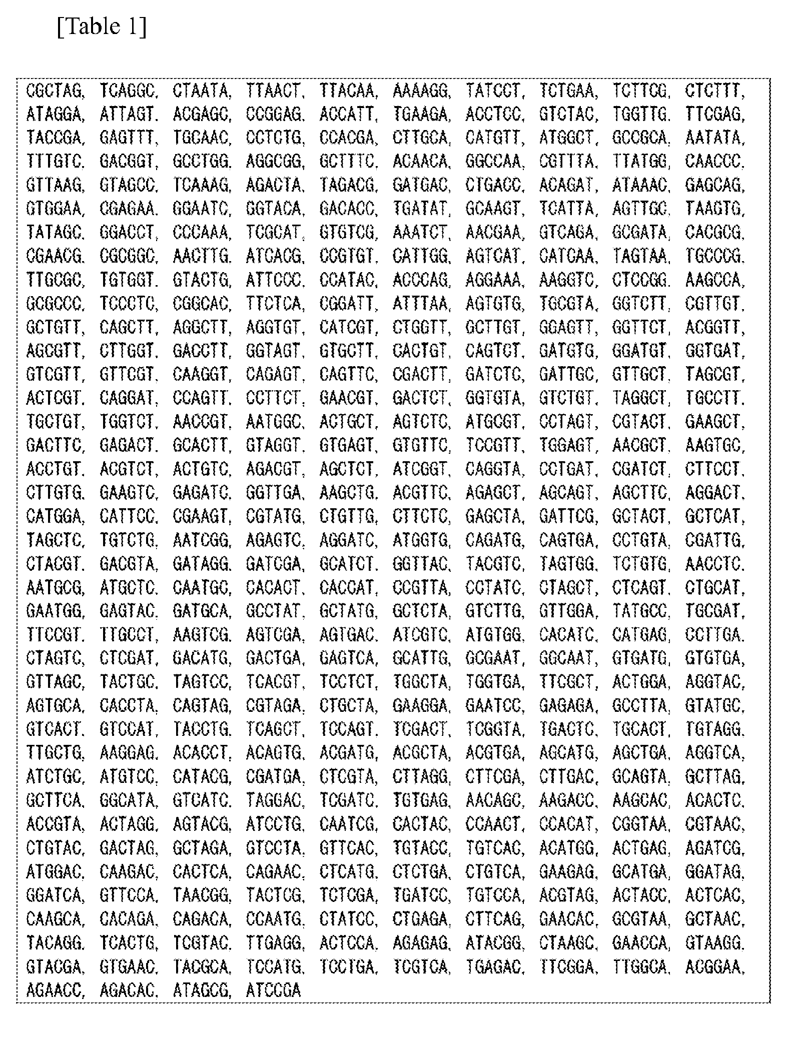

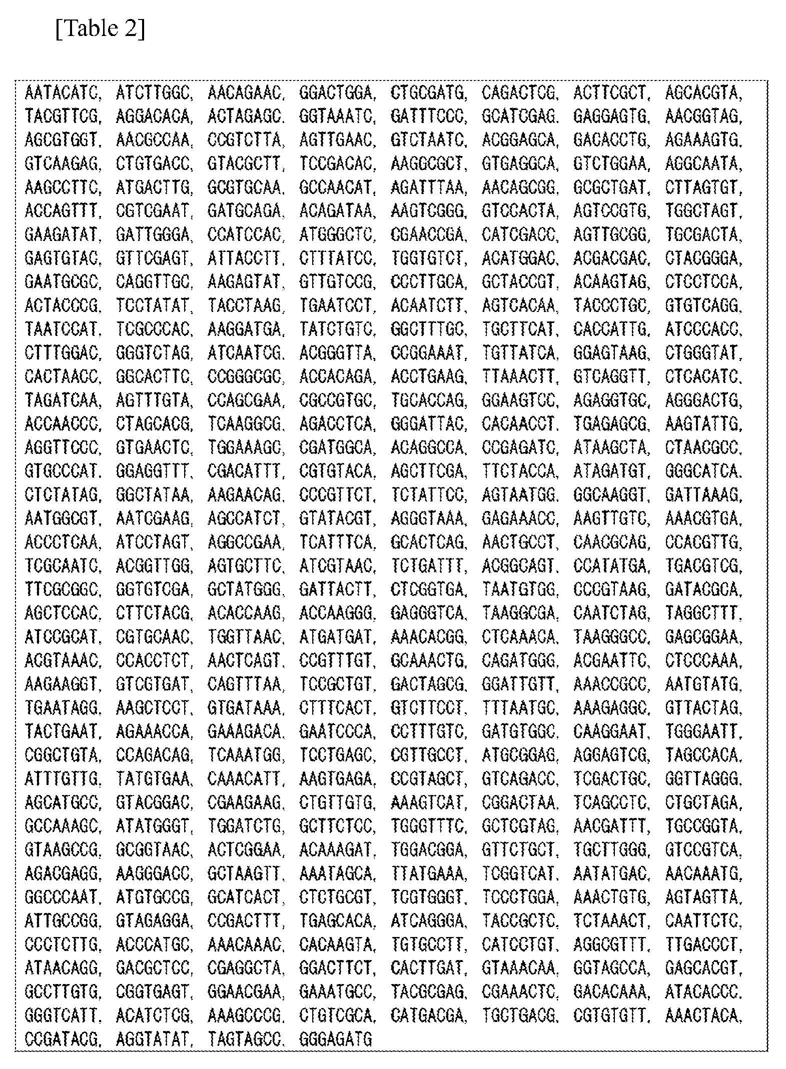

The transposase binding sequence is the same as described above, and examples thereof include a binding sequence such as TN5 transposase, sleeping beauty transposase (SB10), and TN10 transposase.

The caged nucleotide residue is a nucleotide residue that temporarily inactivates the original function by protection with a protective group (caging group) that can be eliminated by irradiation with active energy rays.

The inventors of the present invention found that in a case where a caged nucleotide residue is included in the transposase binding sequence of a nucleic acid fragment having a transposase binding sequence, the transposase cannot be bound.

In addition, it was found that in a case where a caged nucleotide residue contained in a transposase binding sequence is irradiated with active energy rays to eliminate a caging group, the transposase becomes capable of being bound to a transposase binding sequence.

The inventors also found that in a case where a caged nucleotide residue is contained in a polymerase binding sequence of a nucleic acid fragment having a polymerase binding sequence, a polymerase becomes incapable of binding.

In addition, it was found that in a case where a caged nucleotide residue contained in a polymerase binding sequence is irradiated with active energy rays to eliminate a caging group, a polymerase becomes capable of being bound to a polymerase binding sequence.

That is, it can be also said that the present invention provides a method for suppressing binding of a DNA-binding protein to DNA, the method including a step of introducing a caged nucleotide into a binding sequence of the DNA-binding protein on the DNA.

Therefore, for example, in the ChIL method in the related art, by irradiating a region to be reacted with transposase or polymerase with active energy rays at the level of one cell or the level of a sub-cell using a nucleic acid fragment of the present embodiment as a nucleic acid fragment used in the ChIL probe, it became possible to locally carry out the ChIL method only in a region irradiated with active energy rays.

More specifically, in the region irradiated with active energy rays, the caging group is eliminated from a caged nucleotide residue contained in the polymerase binding sequence or the transposase binding sequence, and the polymerase of the transposase becomes capable of binding.

As a result of irradiation with active energy rays, in the region where the transposase is bound to the transposase binding sequence, it is possible to insert a DNA fragment of a desired base sequence in the vicinity of the binding region of the DNA-binding protein bound to the DNA molecule by the ChIL method.

In addition, as a result of irradiation with active energy rays in a region into which a DNA fragment of a desired base sequence is inserted, by the ChIL method, it is possible to perform gene amplification starting from the desired base sequence in a region where a polymerase is bound to a polymerase binding sequence.

Examples of the caging group of the caged nucleotide residue include a 6-nitropiperonyloxymethyl (NPOM) group showing a chemical formula in Formula (1), a 6-nitroveratryloxycarbonyl (NVOC) group showing a chemical formula in Formula (2), a (6-bromo-7-methoxycoumarin)-4-ilmethoxycarbonyl (Bmcmoc) group showing a chemical formula in Formula (3), and the like.

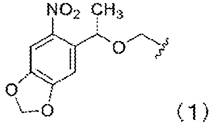

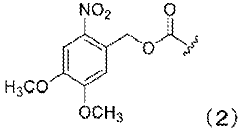

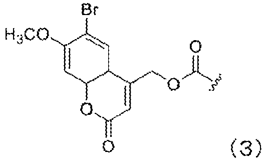

More specific examples of the caged nucleotide residue include an NPOM-dTMP group showing a chemical formula in Formula (4), an NVOC-dCMP group showing a chemical formula in Formula (5), an NVOC-dGMP group showing a chemical formula in Formula (6), a Bmcmoc-dCMP group showing a chemical formula in Formula (7), and the like.

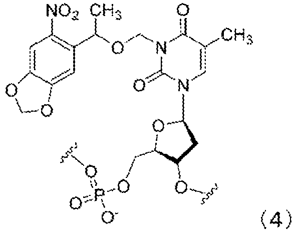

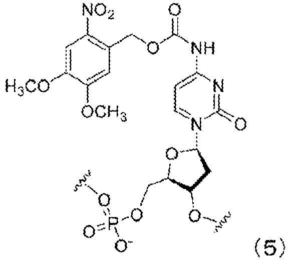

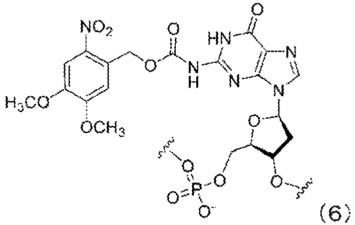

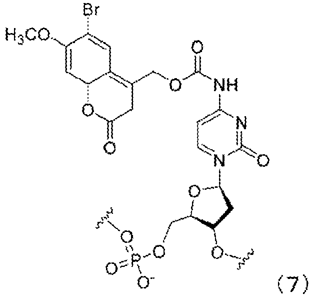

The nucleic acid fragments containing these caged nucleotide residues can be synthesized by chemical synthesis using phosphoramidite corresponding to these caged nucleotide residues as a material.

As the active energy rays, those corresponding to the caging group of the used caged nucleotide residue are used. The active energy rays are not particularly limited as long as they can eliminate the above-described caging group and convert the caged nucleotide residue to the active form, and examples thereof include electromagnetic waves such as ultraviolet rays, visible rays, and infrared rays, α-rays, β-rays, γ-rays, electron beams, neutron beams, radiation such as X-rays, and the like. Among these, light having a wavelength of about 340 to 380 nm is preferable from the viewpoint of not damaging the nucleic acid.

For example, the NPOM group can be eliminated by irradiation with light of about 365 nm. In addition, the NVOC group can be eliminated by irradiation with light of about 350 nm. In addition, the Bmcmoc group can be eliminated by irradiation with light of about 350 nm.

The irradiation with active energy rays can be applied locally to a desired region by using, for example, a confocal laser microscope and the like.

The nucleic acid fragment of the present embodiment is preferably a double-stranded nucleic acid fragment. In a case where the nucleic acid fragment is double-stranded, the charge is neutralized, and thus non-specific binding of the nucleic acid fragment to an unintended location can be suppressed.

It is known that a single-stranded nucleic acid fragment containing one caged nucleotide residue for every five or six residues does not hybridize with a complementary strand. Therefore, the nucleic acid fragment of the present embodiment preferably contains less than one caged nucleotide residue for every five of six residues (single strand) in the polymerase binding sequence or the transposase binding sequence. More specifically, the nucleic acid fragment may contain about 2 to 10 caged nucleotide residues for every 80 residues (single strand).

The nucleic acid fragment of the present embodiment may contain a caged nucleotide residue only in a polymerase binding sequence, may contain a caged nucleotide residue only in a transposase binding sequence, or may contain a caged nucleotide residue in both the polymerase binding sequence and the transposase binding sequence.

In a case where the nucleic acid fragment of the present embodiment contains a caged nucleotide residue in both the polymerase binding sequence and the transposase binding sequence, the caging group of the caged nucleotide residue contained in the polymerase binding sequence and the caging group of the caged nucleotide residue contained in the transposase binding sequence may be different groups.

In addition, irradiation conditions of active energy rays for eliminating the caging group of the caged nucleotide residue contained in the polymerase binding sequence and the caging group of the caged nucleotide residue contained in the transposase binding sequence may be different from each other.

In this case, by controlling the irradiation conditions of active energy rays, it is possible to independently control the caging group of the caged nucleotide residue contained in the polymerase binding sequence and the caging group of the caged nucleotide residue contained in the transposase binding sequence for elimination.

### [Specific binding substance]

In an embodiment, the present invention provides a specific binding substance for a target molecule labeled with the above-described nucleic acid fragment. The specific binding substance of the present embodiment is the same as the above-described conjugate (ChIL probe) except that the polymerase binding sequence or transposase binding sequence contains a caged nucleotide residue.

Examples of the target molecule include a DNA-binding protein or a specific binding substance for the specific binding substance for a DNA-binding protein.

Here, the phrase "a conjugate of the above-described nucleic acid fragment and a specific binding substance for a DNA-binding protein" means a conjugate in which the above-described nucleic acid fragment is bound to a primary antibody, for example. That is, the conjugate may be a conjugate in which the above-described nucleic acid fragment is directly bound to a specific binding substance for a DNA-binding protein.

In addition, the phrase "a conjugate of the above-described nucleic acid fragment and a specific binding substance for the specific binding substance for a DNA-binding protein" means, for example, a conjugate in which the above-described nucleic acid fragment is bound to a secondary antibody. That is, the above-described nucleic acid fragment may not be bound to a specific binding substance for a DNA-binding protein but may be bound to a specific binding substance that is bound to a specific binding substance for a DNA-binding protein. More specifically, in a case where the specific binding substance to the DNA-binding protein is a mouse IgG, examples thereof include a conjugate of the above-described nucleic acid fragment and an anti-mouse IgG antibody and the like.

By using the specific binding substance (ChIL probe) of the present embodiment, it is possible to locally carry out the ChIL method, for example, at the level of one cell or the level of a sub-cell.

### [Method for binding polymerase or transposase to nucleic acid fragment]

In an embodiment, the present invention provides a method for binding a polymerase or transposase to a nucleic acid fragment, including a step of irradiating the above-described nucleic acid fragment with active energy rays to eliminate a caging group from a caged nucleotide residue, and a step of bringing the nucleic acid fragment into contact with a polymerase or a transposase.

By irradiating the above-described nucleic acid fragment with active energy rays, and irradiating the caged nucleotide residue contained in the polymerase binding sequence with active energy rays to eliminate the caging group, it is possible to bind a polymerase to the polymerase binding sequence.

In addition, by irradiating the above-described nucleic acid fragment with active energy rays, and irradiating the caged nucleotide residue contained in the transposase binding sequence with active energy rays to eliminate the caging group, it is possible to bind a transposase to the transposase binding sequence.

According to the method of the present embodiment, it is possible to locally control the binding of the polymerase or transposase to the above-described nucleic acid fragment at the level of one cell or the level of a sub-cell.

### [Method for inserting nucleic acid fragment into vicinity of binding region of DNA-binding protein bound to DNA molecule]

In an embodiment, the present invention provides a method for inserting a nucleic acid fragment containing a polymerase binding sequence and a transposase binding sequence in the vicinity of a binding region of a DNA-binding protein bound to a DNA molecule, in which the transposase binding sequence contains a caged nucleotide residue, the method including a step of bringing the nucleic acid fragment and the binding region close to each other by using a specific binding substance for the DNA-binding protein, a step of irradiating the nucleic acid fragment with active energy rays to eliminate a caging group from the caged nucleotide residue of the transposase binding sequence, a step of binding a transposase to the transposase binding sequence from which the caging group is eliminated, and a step of activating the transposase, and as a result, inserting the nucleic acid fragment in the vicinity of the binding region.

According to the method of the present embodiment, it is possible to locally carry out the ChIL method at the level of one cell or the level of a sub-cell, for example.

In the method of the present embodiment, the nucleic acid fragment may further contain an identification sequence. The polymerase binding sequence, the identification sequence, the transposase binding sequence, the caged nucleotide residue, active energy rays, and the like are the same as those described above.

### [Method for gene-amplifying starting from nucleic acid fragment inserted in vicinity of binding region of DNA-binding protein bound to DNA molecule]

In an embodiment, the present invention provides a method for gene-amplifying a DNA molecule, starting from a polymerase binding sequence, in which a nucleic acid fragment is inserted into the DNA molecule, in which the nucleic acid fragment contains the polymerase binding sequence and a transposase binding sequence, in which the polymerase binding sequence contains a caged nucleotide residue, the method including a step of irradiating the nucleic acid fragment with active energy rays and eliminating a caging group from the caged nucleotide residue of the polymerase binding sequence, and a step of binding a polymerase to the polymerase binding sequence from which the caging group is eliminated to obtain an amplification product.

The method of the present embodiment is a method of performing gene amplification starting from a nucleic acid fragment introduced into a DNA molecule by the above-described method.

According to the method of the present embodiment, it is possible to locally perform gene amplification, for example, starting from a nucleic acid fragment introduced by the ChIL method, for example, at the level of one cell or the level of a sub-cell. Examples of gene amplification include transcription starting from a polymerase binding sequence.

In the method of the present embodiment, the nucleic acid fragment may further contain an identification sequence. The polymerase binding sequence, the identification sequence, the transposase binding sequence, the caged nucleotide residue, active energy rays, and the like are the same as those described above.

### [Examples]

Subsequently, the present invention will be described in more detail with reference to examples, but the present invention is not limited to the following examples.

### [Experimental Example 1]

### (Preparation of ChIL probe containing caged nucleotide residue)

A ChIL probe containing a caged nucleotide residue was prepared.

### <<Preparation of nucleic acid fragment>>

First, a nucleic acid fragment (a base sequence is set forth in SEQ ID NO: 1) containing a polymerase binding sequence, an identification sequence, and a transposase binding sequence in this order was chemically synthesized. An azide group was bound to the 5'-terminal of the nucleic acid fragment of SEQ ID NO: 1. In addition, a complementary strand of the nucleic acid fragment of SEQ ID NO: 1 (each base sequence is set forth in SEQ ID NOS: 2 to 7) was chemically synthesized. A phosphate group was bound to the 5'-terminal of the nucleic acid fragments of SEQ ID NOS: 2 to 7, and TAMRA, which is a fluorescent pigment, was bound to a 3'-terminal.

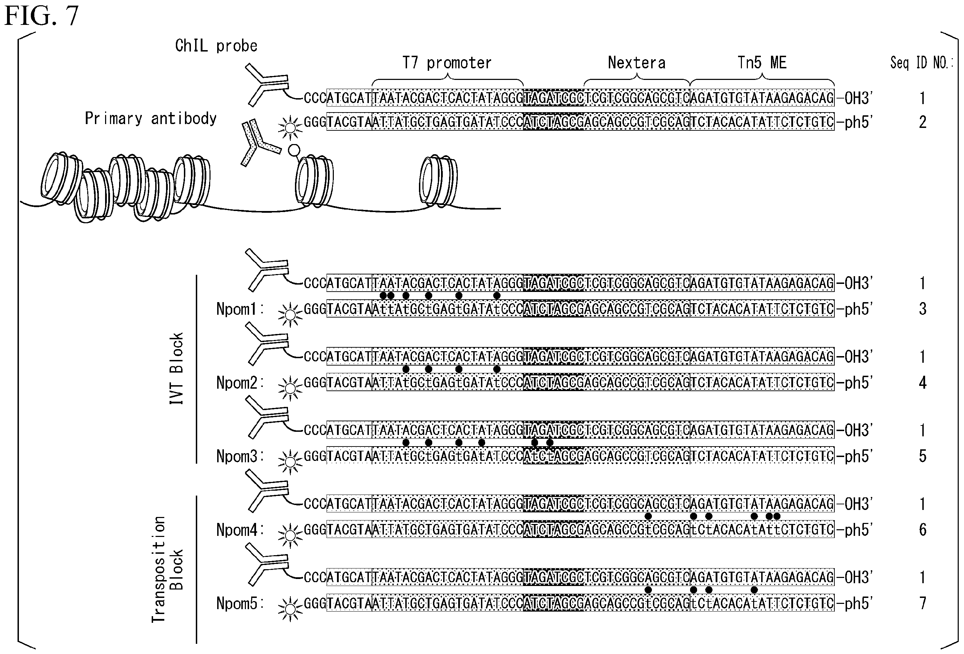

FIG. 7 is a schematic diagram showing the structure of the prepared ChIL probe. In FIG. 7, "primary antibody" indicates a primary antibody, "ChIL probe" indicates a control ChILprobe, and "Npom1" to "Npom5" each indicates a ChILprobe containing a caged nucleotide residue. In addition, the black circles in "Npom1" to "Npom5" indicate positions of the NPOM-dTMP groups. In addition, "T7 promoter" indicates a base sequence of the T7 promoter, "Nextera" indicates an identification sequence for the next generation sequencing, and "Tn5 ME" indicates a Tn5 transposase binding sequence (mosaic end sequence). In addition, it is indicated that an "IVT Block" is for transcriptional inhibition, and a "Transposition Block" is for transposon transposition inhibition. In addition, "OH" indicates a hydroxyl group, and "ph" indicates a phosphate group.

In SEQ ID NO: 1, the polymerase binding sequence was a base sequence of the 10th to 29th positions from the 5'-terminal, and the transposase binding sequence was a base sequence of the 52nd to 70th positions from the 5'-terminal.

In addition, in SEQ ID NO: 3, the residue of the 45th, 50th, 54th, 57th, 59th, and 60th positions from the 5'-terminal was an NPOM-dTMP group. In addition, in SEQ ID NO: 4, the residue of the 45th, 50th, 54th, and 57th positions from the 5'-terminal was an NPOM-dTMP group. In addition, in SEQ ID NO: 5, the residue of the 38th, 40th, 47th, 50th, 54th, and 57th positions from the 5'-terminal was an NPOM-dTMP group. In addition, in SEQ ID NO: 6, the residue of the 8th, 9th, 11th, 17th, 19th, and 25th positions from the 5'-terminal was an NPOM-dTMP group. In addition, in SEQ ID NO: 7, the residue of the 11th, 17th, 19th, and 25th positions from the 5'-terminal was an NPOM-dTMP group.

Subsequently, the nucleic acid fragments of SEQ ID NOS: 1 and 2, the nucleic acid fragments of SEQ ID NOS: 1 and 3, the nucleic acid fragments of SEQ ID NOS: 1 and 4, the nucleic acid fragments of SEQ ID NOS: 1 and 5, the nucleic acid fragments of SEQ ID NOS: 1 and 6, and the nucleic acid fragments of SEQ ID NOS: 1 and 7 were mixed with each other, annealing was performed by heating to 95°C, followed by cooling slowly to form a double-stranded nucleic acid fragment.

### <<Binding of antibody and DBCO-PEG₅>>

An anti-mouse IgG antibody was reacted with dibenzylcyclooctyne (DBCO)-polyethylene glycol (PEG)₅-NHS ester to bind a DBCO group.

### <<Binding of antibody in which DBCO group is bound and double-stranded nucleic acid fragment in which an azide group is bound>>

An anti-mouse IgG antibody to which the DBCO group was bound and each of the above-described double-stranded nucleic acid fragments were mixed with each other, and the two were bound by a click reaction. As a result, each ChIL probe shown in FIG. 7 was obtained.

### [Experimental Example 2]

### (Analysis using ChIL probe containing caged nucleotide residue)

A lysine 27 acetylation modification of a histone H3 in a mouse liver tissue was analyzed using each ChIL probe prepared in Experimental Example 1.

### <<Preparation of tissue section>>

A frozen mouse liver was sliced to a thickness of 10 µm to prepare tissue sections.

### <<Immunostaining>>

A tissue section was lightly washed with phosphate-buffered saline (PBS), 100 µL of a paraformaldehyde (PFA) solution (4% paraformaldehyde/0.3% Triton X-100/PBS) was added thereto, and the mixture was allowed to stand at room temperature for 5 minutes. Subsequently, a PFA solution was removed, and washing was performed three times with 200 µL of a Triton solution (0.3% Triton X-100/PBS). Subsequently, the mixture was allowed to stand in 100 µL of the Triton solution at room temperature until a total of 5 minutes including the time required for the above washing was reached. Subsequently, the Triton solution was removed, and the mixture was washed with 200 µL of PBS.

Subsequently, PBS was removed, 100 µL of a blocking solution (product name "Blocking One", catalog number "# 03953-66", Nacalai Tesque, Inc.) was added thereto, and the mixture was allowed to stand at room temperature for 20 minutes. Subsequently, the blocking solution was removed, and washing was performed with 200 µL of PBS.

Subsequently, PBS was removed, M. O. M IgG blocking solution (3.6 µL M. O. M IgG blocking (Vector Laboratories, Inc.)/100 µL of PBS) was added, and the mixture was allowed to stand for 1 hour.

Subsequently, the solution was removed, the mixture was washed once with PBS, 100 µL of a primary antibody diluted with M. O. M diluent (2.6 µL M. O. M protein concentrate (Vector Laboratories, Inc.)/PBS) was added thereto, and the mixture was allowed to stand at 4°C overnight. As the primary antibody, a mouse IgG antibody (hereinafter, may be referred to as "anti-H3K27ac antibody") against a lysine 27 acetylation modified histone H3 was used. Subsequently, the primary antibody solution was removed, and washing was performed three times at room temperature using 200 µL of PBS.

Subsequently, PBS was removed, and 100 µL of each ChIL probe solution (1:100) diluted with M. O. M diluent (2.6 µL MOM protein concentrate (Vector Laboratories, Inc.)/0.5 M NaCl/PBS)) was added. Subsequently, a lid was covered and sealed to prevent evaporation, in a case of performing fluorescence observation, light was further shielded with aluminum foil, and the mixture was allowed to stand at 4°C overnight.

As a result, a DNA fragment labeled with the ChIL probe and a binding region of the lysine 27 acetylation modified histone H3 on a genomic DNA were brought close to each other. Subsequently, the ChIL probe solution was removed, and washing was performed three times at room temperature using 200 µL of PBS.

Subsequently, 200 µL of PBS was added thereto, and a stained image with a ChIL probe was observed with a fluorescence microscope. Subsequently, a target area was irradiated with ultraviolet rays (wavelength 355 nm) for 15 minutes. The target area contained approximately 10,000 cells. As a result, in the ChIL probe in the ultraviolet-irradiated region, the NPOM group was eliminated from the NPOM-dTMP group, and a polymerase or transposase became capable of binding.

### <<ChIL reaction>>

PBS was removed, 100 µL of a Tn5 solution (150 ng/well in 1 × Tn5 dialysis buffer) was added thereto, and the mixture was allowed to stand at room temperature for 1 hour. The composition of the 1 × Tn5 dialysis buffer was formed of 50 mM HEPES-KOH (pH 7.2), 0.1 M NaCl, 0.1 mM EDTA, 1 mM DTT, 0.1% Triton X-100, and 10% glycerol. As a result, the transposase was bound to the transposase binding sequence of the double-stranded nucleic acid fragment of each ChIL probe.

Subsequently, a free DNA fragment having a transposase binding sequence was added to a sample, and the mixture was allowed to stand at room temperature for 1 hour. The free DNA fragment having a transposase binding sequence was obtained by annealing a DNA fragment consisting of a base sequence set forth in SEQ ID NO: 8 and a DNA fragment consisting of a base sequence set forth in SEQ ID NO: 9. Subsequently, washing was performed three times at room temperature using 200 µL of PBS. Subsequently, 100 µL of 1 × Tn5 dialysis buffer was added thereto to replace the buffer.

Subsequently, the 1 × Tn5 dialysis buffer was removed, 100 µL of a 1 × TAPS-DMF buffer (10 mM TAPS-NaOH (pH 8.5), 5 mM MgCl₂, 10% N,N-dimethylformamide) was added thereto, and the mixture was allowed to stand at 37°C for 1 hour. With this, the transposase bound to the double-stranded nucleic acid fragment of the ChIL probe was activated, and the double-stranded nucleic acid fragment of the ChIL probe was inserted in the vicinity of the binding region of the lysine 27 acetylation modified histone H3 on the genomic DNA.

Subsequently, 1 × TAPS-DMF buffer was removed, 100 µL of a 0.2% sodium dodecyl sulfate (SDS) solution was added thereto, and the mixture was allowed to stand at room temperature for 10 minutes. Subsequently, the SDS solution was removed, and washing was performed three times at room temperature using 200 µL of PBS. With this, the transposase was deactivated and removed.

Subsequently, the PBS was removed, and 100 µL of 1 × T4 DNA ligase buffer was added thereto to replace the buffer. Subsequently, a fill-in solution having the composition shown in Table 3 below was prepared, added to a well from which a 1 × T4 DNA ligase buffer was removed, and allowed to stand at room temperature for 1 hour. With this, a double-stranded nucleic acid fragment of the ChIL probe was reliably inserted in the vicinity of the binding region of the lysine 27 acetylation modified histone H3 on the genomic DNA.

**[Table 3]**

| Fill-in solution | |
|---|---|
| Nuclease-free water | 88.5 µL |
| 10 × T4 DNA ligase buffer | 10 µL |
| dNTP mix (each 10 mM) | 0.5 µL |
| T4 DNA ligase (400 U/µL) | 0.5 µL |
| T4 DNA polymerase (3 U/µL) | 0.5 µL |
| Total | 100 µL (per 1 well) |

Subsequently, the fill-in solution was removed, 100 µL of a 0.2% sodium dodecyl sulfate (SDS) solution was added, and the mixture was allowed to stand at room temperature for 10 minutes. Subsequently, the SDS solution was removed, and washing was performed three times at room temperature using 200 µL of PBS. As a result, T4 DNA ligase and T4 DNA polymerase were deactivated and removed.

Subsequently, the PBS was removed, and 100 µL of 1 × T7 RNA polymerase buffer was added thereto to replace the buffer. Subsequently, an in-situ transcription reaction solution having the composition shown in Table 4 below was prepared, and added to a well from which a 1 × T7 RNA polymerase buffer was removed. Subsequently, a lid was covered and sealed to prevent evaporation, and the mixture was allowed to stand at 37°C overnight. As a result, the genomic DNA was transcribed (hereinafter, the transcribed RNA may be referred to as "ChIL RNA") starting from the T7 promoter sequence contained in the inserted DNA fragment.

**[Table 4]**

| In-situ transcription reaction solution | |
|---|---|
| 0.05% Tween20/nuclease-free water | 69 µL |
| 10 × T7 RNA polymerase buffer | 8 µL |
| 100 mM ATP | 0.5 µL |
| 100 mM GTP | 0.5 µL |
| 100 mM CTP | 0.5 µL |
| 100 mM UTP | 0.5 µL |
| RNase inhibitor (40 U/µL) | 0.5 µL |
| T7 RNA polymerase (200 U) | 0.5 µL |
| Total | 80 µL (per 1 well) |

Subsequently, 0.5 µL of DNaseI (catalog number "2270A", Takara Bio Inc.) was added thereto, and the mixture was allowed to stand at 37°C for 30 minutes. As a result, the DNA in the sample was degraded. Subsequently, each ChIL RNA was purified with 1.8 times the volume of Agencourt RNAClean XP beads (catalog number "# A63987", Beckman Coulter, Inc.) and eluted with 10 µL of nuclease-free water. The beads were removed.

### <<Preparation of sequencing library>>

A primer solution for reverse transcription having the composition shown in Table 5 below was prepared. All preparations for enzymatic reaction were performed on ice.

**[Table 5]**

| Primer solution for reverse transcription (SMART-seq v4 kit, Clontech, Inc.) | |
|---|---|
| 10 × Lysis buffer | 4.75 µL |
| PCR Primer II A (12 µM) | 5 µL |
| RNase inhibitor (40 U/µL) | 0.25 µL |
| T7 RNA polymerase (200 U) | 0.5 µL |
| Total | 10 µL (per 10 samples) |

Subsequently, each ChIL RNA and a primer solution for reverse transcription were added and suspended in a 0.2 mL tube at the volume shown in Table 6 below.

**[Table 6]**

| | |
|---|---|
| ChIL RNA | 5.25 µL |
| Primer solution for reverse transcription | 1 µL |
| Total | 6.25 µL (per 1 sample) |

Subsequently, after performing heating with a thermal cycler at 72°C for 3 minutes, the mixture was immediately put back on ice. Subsequently, a reverse transcription reaction solution having the composition shown in Table 7 below was prepared, added to each sample, and mixed. Subsequently, after reaction at 42°C for 90 minutes using a thermal cycler, reaction was performed at 70°C for 10 minutes, and the resultant product was allowed to stand at 4°C. As a result, each ChIL RNA was reverse transcribed, and a ChIL cDNA was obtained.

**[Table 7]**

| Reverse transcription reaction solution (SMART-seq v4 kit, Clontech, Inc.) | |
|---|---|
| 5 × Ultra Low First-Strand buffer | 2 µL |
| SMART-Seq v4 oligonucleotide | 0.5 µL |
| RNase inhibitor (40 U/µL) | 0.25 µL |
| SMARTScribe reverse transcription enzyme | 1 µL |
| Total | 3.75 µL (per 1 sample) |

Subsequently, a PCR reaction solution having the compositions shown in Table 8 below was prepared. An Ad1 primer is a primer that is partially complementary to the nucleic acid fragment of the ChIL probe. A base sequence of the Ad1 primer is set forth in SEQ ID NO: 10.

**[Table 8]**

| PCR reaction solution (SMART-seq v4 kit, Clontech, Inc.) | |
|---|---|
| 2 × SeqAmp PCR buffer | 12.5 µL |
| Ad1 primer (10 µM, SEQ ID NO: 10) | 0.5 µL |
| SeqAmp DNA polymerase | 0.5 µL |
| Nuclease-free water | 1 µL |
| Total | 14.5 µL (per 1 sample) |

Subsequently, each of the PCR reaction solution, each ChIL cDNA, and an Ad2 primer was mixed at the volume shown in Table 9 below. The Ad2 primer is a primer that is partially complementary to a primer for reverse transcription. The base sequence of the Ad2 primer is set forth in SEQ ID NO: 11.

**[Table 9]**

| | |
|---|---|
| ChIL cDNA | 10 µL |
| Ad2 primer (10 µM, SEQ ID NO: 11) | 0.5 µL |
| PCR reaction solution | 14.5 µL |
| Total | 25 µL (per 1 sample) |

Subsequently, a PCR reaction was performed using a program shown in Table 10 below using a thermal cycler.

**[Table 10]**

| | | |
|---|---|---|
| 95°C | For 1 minute | 1 cycle |
| 98°C | For 10 seconds | 15 cycles |
| 65°C | For 30 seconds | |
| 68°C | For 3 minutes | |
| 72°C | For 10 minutes | 1 cycle |

Subsequently, purification was performed with a DNA Cleanup Concentrator (ZYMO RESEARCH). Subsequently, purification was performed twice with an equal volume of AMPure XP beads (catalog number "#A63881", Beckman Coulter, Inc.) and eluted with 15 µL of 10 mM Tris buffer or nuclease-free water to obtain each sequencing library. The beads were removed.

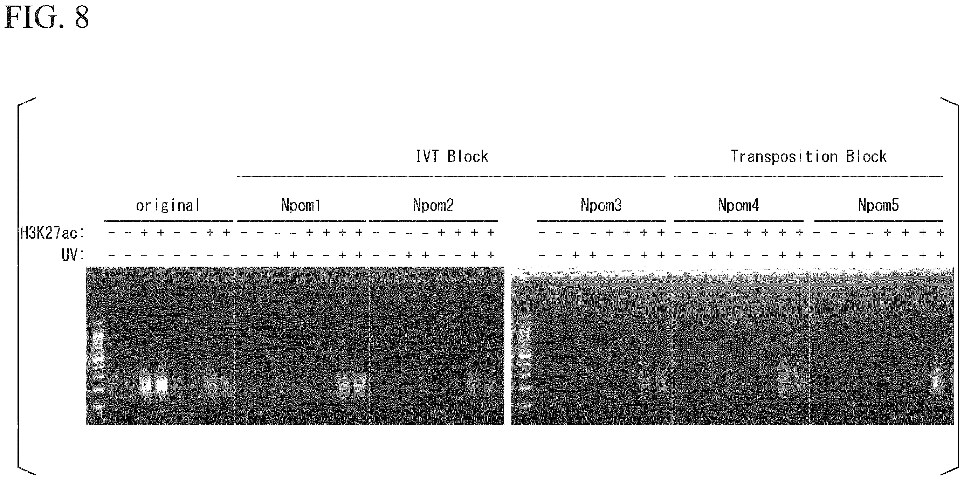

FIG. 8 is a photograph showing results of confirming each sequencing library by agarose electrophoresis. In FIG. 8, "H3K27ac" indicates an anti-H3K27ac antibody used for a primary antibody, "UV" indicates ultraviolet rays, "+" indicates that the anti-H3K27ac antibody was added or irradiated with ultraviolet rays, and "-" indicates that the anti-H3K27ac antibody was not added or not irradiated with ultraviolet rays. In addition, "original" indicates results obtained by using a control ChIL probe, and "Npom1" to "Npom5" each indicates a ChIL probe containing a caged nucleotide residue, which was prepared in Experimental Example 1. In addition, "IVT Block" indicates results obtained by using the ChIL probe for transcriptional inhibition, and "Transposition Block" indicates results obtained by using the ChIL probe for transposon transposition inhibition.

As a result, in a case where an anti-H3K27ac antibody was added and the caging group was eliminated by irradiation with UV in a sequencing library using Npom1 to 5, a DNA band, which was the same size as the sequencing library obtained by using the control ChIL probe, was strongly detected near 200 to 400 bp. On the other hand, in a case where the H3K27ac antibody was added and UV irradiation was not performed, in a case where the H3K27ac antibody was not added and UV irradiation was performed, and in a case where the H3K27ac antibody was not added and UV irradiation was not performed, it was clarified that the DNA band of the sequencing library was barely detected.

This result indicates that the caging group was eliminated by UV irradiation, the ChIL reaction was enabled, and the sequencing library could be prepared.

Subsequently, each sequencing library prepared by performing UV irradiation was sequenced (hereinafter, may be referred to as "ChlL-seq"). NovaSeq6000 (Illumina, Inc.) was used as the sequencer, and sequencing was performed in 100 cycles using an SP flow cell. Subsequently, only a read sequence valid for analysis was extracted from the obtained sequence data, and the data quality was evaluated.

Specifically, visualization was performed by Integrative Genomics viewer (IGV) in order to confirm whether or not an appropriate signal was obtained in ChlL-seq using Npom1 to 5.

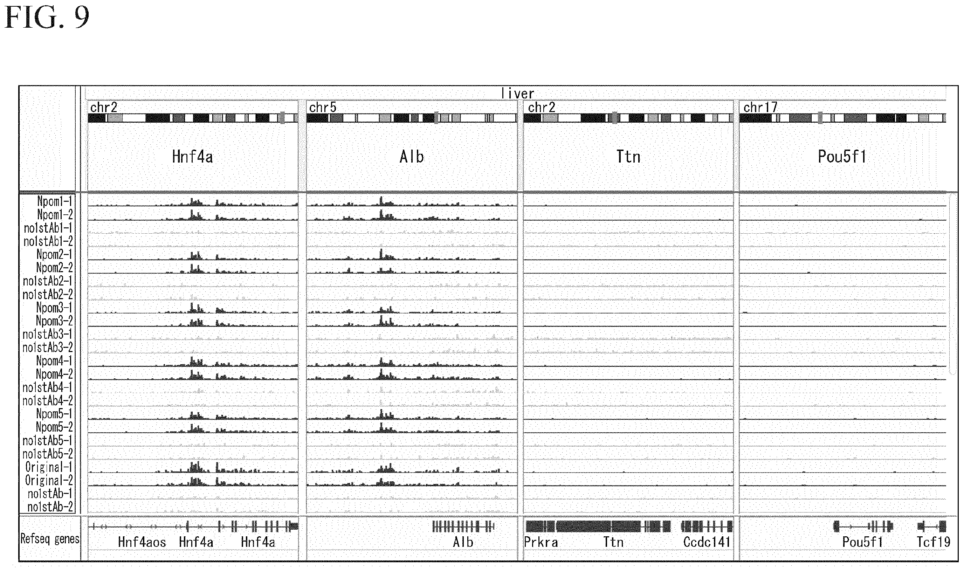

FIG. 9 is a diagram showing a result of the visualization. In FIG. 9, a vertical axis indicates signal intensity and a horizontal axis indicates genome position information. In addition, "Npom1-1" and "Npom1-2" indicate results of ChIL-seq using Npom1, "no1stAb1-1" and "no1stAb1-2" indicate results of ChlL-seq in which Npom1 was reacted without adding a primary antibody, "Npom2-1" and "Npom2-2" indicate results of ChlL-seq using Npom2, "no1stAb2-1" and "no1stAb2-2" indicate results of ChlL-seq in which Npom2 was reacted without adding the primary antibody, "Npom3-1" and "Npom3-2" indicate results of ChlL-seq using Npom3, "no1stAb3-1" and "no1stAb3-2" indicate results of ChlL-seq in which Npom3 was reacted without adding the primary antibody, "Npom4-1" and "Npom4-2" indicate results of ChlL-seq using Npom4, "no1stAb4-1" and "no1stAb4-2" indicate results of ChlL-seq in which Npom4 was reacted without adding the primary antibody, "Npom5-1" and "Npom5-2" indicate results of ChlL-seq using Npom5, "no1stAb5-1" and "no1stAb5-2" indicate results of ChlL-seq in which Npom5 was reacted without adding the primary antibody, "Original-1" and "Original-2" indicate results of ChlL-seq using a control ChIL probe, and "no1stAb-1" and "no1stAb-2" indicate results of ChlL-seq in which the control ChIL probe was reacted without adding the primary antibody. In addition, "Hnf4a" and "Alb" are genes that are highly expressed in the liver, and "Ttn" and "Pou5f1" are genes that are little expressed in the liver.

As a result, in the ChlL-seq using Npom1 to 5, H3K27ac-modified signals were detected in the Hnf4a gene and the Alb gene, which are highly expressed in the liver. On the other hand, in the Ttn gene and the Pou5f1 gene, which are little expressed in the liver, H3K27ac-modified signals were not detected.

In addition, a pattern of the H3K27ac-modified signals in the ChlL-seq using Npom1 to 5 was equivalent to a pattern of the H3K27ac-modified signals in the ChIL-seq using a control ChIL probe that did not require UV irradiation.

The above results indicate that it is possible to locally perform ChIL-seq by performing UV irradiation using Npom1 to 5.

### [Industrial Applicability]

According to the present invention, it is possible to provide a simpler technique capable of locally analyzing a base sequence of a target nucleic acid, for example, at the level of one cell or at the level of a sub-cell.

### [Reference Signs List]

10: Genomic DNA
10': Complementary strand of genomic DNA 10
11: Binding region
20: Histone
30: Specific binding substance
40: DNA fragment
41: Transposase binding sequence
41': Complementary strand of transposase binding sequence 41
42: T7 promoter sequence
43: Identification sequence
43': Complementary strand of identification sequence 43
44: Linker
44': Base sequence derived from part of linker 44
45: Optional sequence
50: Transposase
60: RNA

## Claims

1. A nucleic acid fragment, comprising:
a polymerase binding sequence; and
a transposase binding sequence,
wherein the polymerase binding sequence or the transposase binding sequence contains a caged nucleotide residue.

2. The nucleic acid fragment according to Claim 1, which is a double-stranded nucleic acid fragment.

3. The nucleic acid fragment according to Claim 1 or 2,
wherein a caging group of the caged nucleotide residue contained in the polymerase binding sequence and a caging group of the caged nucleotide residue contained in the transposase binding sequence are different groups.

4. A specific binding substance for a target molecule labeled with the nucleic acid fragment according to any one of Claims 1 to 3.

5. A method for binding a polymerase or a transposase to the nucleic acid fragment according to any one of Claims 1 to 3, the method comprising:
a step of irradiating the nucleic acid fragment with active energy rays and eliminating a caging group from the caged nucleotide residue; and
a step of bringing the polymerase or the transposase into contact with the nucleic acid fragment.

6. A method for inserting a nucleic acid fragment containing a polymerase binding sequence and a transposase binding sequence in a vicinity of a binding region of a DNA-binding protein bound to a DNA molecule, the transposase binding sequence containing a caged nucleotide residue, the method comprising:
a step of bringing the nucleic acid fragment and the binding region close to each other by using a specific binding substance for the DNA-binding protein;
a step of irradiating the nucleic acid fragment with active energy rays and eliminating a caging group from the caged nucleotide residue of the transposase binding sequence;
a step of binding a transposase to the transposase binding sequence from which the caging group is eliminated; and
a step of activating the transposase, and as a result, inserting the nucleic acid fragment in the vicinity of the binding region.

7. A method for gene-amplifying a DNA molecule, starting from a polymerase binding sequence, wherein a nucleic acid fragment is inserted into the DNA molecule, wherein the nucleic acid fragment contains the polymerase binding sequence and a transposase binding sequence, in which the polymerase binding sequence contains a caged nucleotide residue, the method comprising:
a step of irradiating the nucleic acid fragment with active energy rays and eliminating a caging group from the caged nucleotide residue of the polymerase binding sequence; and
a step of binding a polymerase to the polymerase binding sequence from which the caging group is eliminated to obtain an amplification product.
